# EUROPEAN PATENT APPLICATION

(11) **EP 1 169 995 A1**
(43) Date of publication of application: **09.01.2002**
(21) Application number: 00202423.0
(22) Date of filing: 07.07.2000
(51) Int. Cl.: A61K 6/00, C08F 220/00

(54) **Adhesive for removable prosthesis**

(71) Applicant: Universiteit Utrecht, 3584 CX Utrecht (NL)
(72) Inventor: De Putter, Cornelis, 3604 AB Maarssen (NL); Hinrichs, Wouter Leonardus, 9718 EG Groningen (NL); Hennink, Wilhelmus Everhardus, 2743 CZ Waddinxveen (NL)
(74) Representative: Prins, Adrianus Willem

(57) **Abstract**

The invention relates to a thermosensitive adhesive composition comprising:
a) a first monomer chosen from the group of alkyl acrylates and alkyl methacrylates containing in the alkyl group 4 to 10 carbon atoms;
b) a second monomer chosen from the group of alkyl acrylates or methacrylates containing in the alkyl group 1 to 3 carbon atoms, acrylamides, methacrylamides and other unsaturated monomers; and
c) 1 to 15 mole% of acrylic acid, methacrylic acid and/or hydroxyethyl methacrylic acid,
which copolymer has a glass transition temperature between 45 and 70°C.

## Description

The invention relates to an adhesive composition for removable prostheses, particularly dental prostheses, and the use of said composition adhering removable prosthesis of various types to a patient's epithelium, mucosa or skin.

In dentistry, replacement of teeth that are extracted or lost due to, mostly, periodontal disease, caries or trauma, can be performed with full dentures, partial dentures, bridges or implants. Finding retention for prosthetic appliances is one of the greatest difficulties in prosthetic dentistry. This is particularly a major problem in edentulous cases where resorption of the alveolar bony ridge has taken place to such an extent, that finding retention for a removable denture is greatly compromised.

The most common complaint of full denture wearers is looseness of their dentures or fear for that. Full dentures are retained in the mouth by creating vacuum forces and by support from the upper and lower jaws. Removable partial dentures also derive retention from the remaining natural teeth. Fixed bridges derive retention only from the remaining natural teeth.

With fixation to natural teeth, maximal retention is attained, since natural teeth are retained by fixation of their roots in the alveolar bone of upper and lower jaws. When natural teeth are missing, retention for prosthetic appliances can be sought in the jaw bone with dental implants. For placement of these implants, however, sufficient jaw bone must still be present or be transplanted to the implantation area. In addition to that, the patient must be in sufficient health for this type of surgical procedure, be willing to undergo it and must be capable to deal with the risks and often high financial costs involved. When one or more of these requirements cannot be fulfilled, especially edentulous patients often use adhesives for their removable full dentures with the purpose to avoid functional problems with chewing, speaking, laughing and kissing.

There are several products on the market that are to be used as adhesives for dental prostheses. These products are generally in the form of a glue that needs to be applied afresh every time the prosthesis is reinserted into the oral cavity. A disadvantage of the known products is that their performance hardly comes close to the delicate balance between a firm attachment in the oral cavity on the one hand, and an easy release when the prosthesis is to be taken out on the other hand.

Accordingly, there is a need for an adhesive which provides a firm attachment of a dental prosthesis in the oral cavity, but of which the adhesive strength can be broken, or switched off, when the prosthesis is to be taken out. In other words, the adhesive should not lead to any risk of undesired movement or release of the prosthesis when it is to remain in place, but should allow for an easy and convenient release of the prosthesis should its user so desire.

In a first embodiment, the present invention provides a thermosensitive adhesive for prostheses. By the term 'thermosensitive' is meant that the adhesive strength of the substance varies with the temperature. Conveniently, the substance may for instance provide strong attachment and adhesive strength below a certain temperature, but essentially no adhesive strength above said temperature.

Preferably, the present adhesive is based on a specific copolymer. The term copolymer as used herein is intended to encompass all polymers that are prepared from more than one type of monomer. Thus, terpolymers and the like, which are based on three or more types of monomers are to be comprised by the term copolymer.

The specific copolymer comprises a first monomer chosen from the group of alkyl acrylates and alkyl methacrylate containing in the alkyl group 4 to 10 carbon atoms, preferably 6 to 10 carbon atoms, more preferably 6 to 8 carbon atoms, and most preferably 8 carbon atoms. Examples of suitable alkyl acrylates or methacrylates are n-butyl, n-pentyl, n-hexyl, isoheptyl, n-nonyl, n-decyl, isohexyl, 2-ethyloctyl, isooctyl and 2-ethylhexyl acrylates or methacrylates, and combinations thereof. In general, methacrylates are preferred. The most preferred methacrylate is 2-ethylhexyl methacrylate.

The copolymer further comprises a second monomer chosen from the group of monomers which form copolymers with monomers of the first group. Such monomers are alkyl acrylates or methacrylates containing in the alkyl group 1 to 3 carbon atoms, acrylamides, methacrylamides and other unsaturated monomers. Suitable examples to be used as the second monomer include acrylamide, N-vinyl-2-pyrrolidone, vinyl acetate, N-isopropyl acrylamide, and methyl methacrylate, and combinations thereof. Preferably, methylmethacrylate is used.
An important parameter of the copolymer is its glass transition temperature. This parameter can conveniently be adjusted by the skilled person by choosing suitable monomers in suitable amounts, within the ranges set forth above. It is preferred that the glass transition temperature of the copolymer lies between-25 and 20°C, more preferably between -15 and 10°C. In accordance with the invention The glass transition temperature (Tg) of the different copolymer may be determined by Modulated Differential Scanning Calorimetry (MDSC). MDSC measurements are carried out with a DSC 2920 differential scanning calorimeter (TA Instruments, New Castle, England). Temperature and heat flow calibration may be performed with indium. For the MDSC analysis, the samples (round 5-10 mg) may first be cooled to -100 °C for 5 minutes and then heated to 100 °C in a stream of helium. The heating rate is preferably 2°C/min, the amplitude 0.318 °C, and the period 60 seconds.

The glass transition temperature can conveniently be achieved by choosing suitable monomers in suitable amounts for the copolymer.

Generally, it is preferred that the molar ratio of the first monomer to the second monomer is between 1:1 and 3:1. In a highly preferred embodiment, the copolymer comprises between 1 to 90 mole%, more preferably between 50 and 70 mole%, even more preferably between 55 and 65 mole% of the first monomer. The second monomer is preferably present in an amount between 1 and 90 mole%, more preferably between 25 and 45 mole%, even more preferably between 30 and 40 mole%, in the copolymer.

The copolymer also comprises 1 to 15 mole%, preferably 2 to 8 mole% of a compound which ensures a good adherence to living tissue. Examples of such compounds include acrylic acid, methacrylic acid, hydroxyethyl methacrylic acid (HEMA) succinylated HEMA, and combinations thereof.

Preferably, the copolymer has a relatively low molecular weight. In principle, the molecular weight should be low enough to attain good flow properties and high enough to attain a good internal cohesion of the copolymer. Good results have been obtained with copolymers having a weight average molecular weight (M_{w}) between 1,000 and 200,000, preferably between 2,000 and 25,000. The average molecular weight (M_{w}, Mₙ) can suitably be determined using gel permeation chromatography (GPC). This technique, which is known per se, may for instance be performed using tetrahydrofuran as a solvent and polystyrene with different molecular weights as calibration standards. The ratio between the weight and number average molecular weights of the copolymer will have a value which is typical for these types of copolymers prepared by normal radical polymerization. Exemplary values lie between 2 and 20.

The molecular weight of the copolymer can easily be controlled using standard measures known in the art. A suitable manner of controlling the molecular weight is by use of a chain transfer agent. Various chain transfer agents known in the art may be employed. These include mercaptans, such as mercaptoethanol, mercaptoacetic acid, mercaptopropionic acid, and the like. The amount of chain transfer agent needed can easily be determined on the basis of the objective molecular weight of the copolymer on the basis of the normal knowledge of the skilled artisan.

The preparation of the copolymer may be carried out in any known manner, for instance through the mechanism of radical polymerization as described in WO-A-95/15754 or US-Re-24,906. The polymerization can be performed using a free radical initiator such as an organic peroxide, e.g. benzoylperoxide, or an organic azo compound, such as 2,2'-azobis(2,4-dimethylpentanenitrile) or 2,2'-azobisisobutyronitrile. Other types of polymerization, such as solution, suspension or emulsion polymerization may also be used.

Care should be taken after the polymerization reaction that essentially all unreacted monomers, initiator and fragments thereof, and unreacted chain transfer agent are removed from the copolymer. A suitable manner to accomplish this is by re-precipitation.

A thermosensitive adhesive in accordance with the invention can optionally comprise conventional additives. As one of the more important intended applications of the adhesive is in the field of dentistry, it may be desired to add flavoring and/or sweetening agents. Other additives that may be employed include antimicrobial agents (antibiotics), inflammation inhibitors and other drugs, antioxidants, pigments, and the like.

A preferred form wherein a thermosensitive adhesive according to the invention may be used is an aqueous dispersion. On the one hand, the concentration of the copolymer in the dispersion is preferably as high as possible. On the other hand, said concentration should not be too high, as this might lead to poor flow properties making the adhesive more difficult to handle. On the basis of this consideration, the skilled person will be able to select a suitable concentration.

In a second embodiment, the invention is related to the use of the above described adhesive for adhering a prosthesis to epithelium, mucosa or skin. In the context of the present invention, a prosthesis is understood to be an artificial, non-living element with which a patient is to be provided in replacement with a lost or partly lost organ or body part. Particularly advantageous is the use of the adhesive for a dental prosthesis, replacing one, more or all teeth and/or their supporting tissues when they are lost and have to be replaced to restore oral functions, esthetics and comfort.

The present adhesive has been found to be particularly suitable for adhering such products to epithelial tissue. Examples of prostheses that may be adhered are not only in the field of dental elements, but also encompass artifical noses or ears.

In order to adhere a prosthesis to human epithelium, the adhesive may be applied to either the prosthesis, the epithelium or both. Preferably, the adhesive is only applied to the prosthesis' surface that is to be adhered to the epithelium. To this end, the adhesive may be applied every time the prosthesis has been released and needs to be re-attached, for instance from a tube.

However, as it is one of the great advantages of the present adhesive that its adhering power can be 'switched on and off at will, it can be re-used. Therefore, in a preferred embodiment, a surface of a prosthesis, or a part thereof, may be provided with the present adhesive, e.g. in the form of an adhesive strip. This strip will adhere to human epithelium at body temperature, i.e. around 37°C, but may come loose upon cooling or heating. Preferably, the adhesive will release at a temperature between 45-60°C, more preferably between 50-55°C. It has been found that such temperatures are agreeable when applied to the oral cavity. Surprisingly, the adhesive has such adhesive strength that drinking a hot beverage will not be sufficient to release the prosthesis. Instead, a heating applicator may be used to apply the required temperature to the prosthesis, in particular to the adhering surface thereof, for a sufficient time to 'switch off the adhesive.

The invention will now be elucidated by the following, non-restrictive examples.

### EXAMPLE I

In a flask, 2.68 g of methyl methacrylate (MMA), 12.63 grams of 2-ethylhexyl methacrylate (EHMA) were dissolved in 50 mL of toluene under an atmosphere of dry nitrogen at 60°C. The ratio MMA/EHMA was 17.5/82.5 (w/w). To the solution, 155.5 mg of methacrylic acid (MAA) was added (2 mole%). Upon addition of 148 mg of 2,2'-azobisisobutyronitrile (AIBN), which corresponds with 1 mole% of initiator with respect to the total amount of monomers, and 456 mg of mercaptoethanol as chain transfer agent (CTA), which corresponds with 12.5 mole% of initiator with respect to the total amount of monomers, polymerization started. The reaction was completed after stirring at 60°C for 24 hours.

After polymerization, about half of the solvent was removed using a rotavap. Subsequently, the product was precipitated by adding the solution dropwise to a large excess of methanol at -20 à -30°C. After drying, the product was dissolved in about 15 mL of toluene and precipitated again by dropwise addition of the solution to a large excess of methanol at -20 à -30°C. To remove remnants of toluene and methanol, the product was exposed to air at ambient temperature for two days, and thereafter kept in a vacuum oven until constant weight (three days). The temperature of the vacuum oven was gradually increased from ambient temperature to 75°C. The yield was about 75%. In the ¹H-NMR spectrum of the product, no monomer residues could be detected (detection level ca. 0.5%).

The adhesive strength of the copolymer was determined at different temperatures. This was done using a post mortem prepared dog jaw, which had been deep frozen and defrosted. On this jaw, a circular press tool (or stamp) having a diameter of 2.7 cm was prepared using an imprint. This press tool was attached to the jaw using one of the tested adhesive compositions, after which a tension test was performed. The results are shown in table 1. As a reference, it can be noted that the adhesive strength of commercial dental adhesives at 37°C was determined using the same technique to be between 0.4 and 5 N.

**Table 1:**

| Adhesive strength at different temperatures | |
|---|---|
| Temperature (°C) | Adhesive strength (N) |
| 14 | 30 |
| 37 | 23 |
| 60 | 9 |

It was found that an adhesive strength of 9 N can easily be overcome. In other words, a prosthesis adhered to epithelium using the tested copolymer can easily be removed manually at temperatures around 60°C.

### Example II

The following polymers were synthesized via essentially the same procedure as described in Example I:

**Table 2:**

| Data of polymers | | | | | | | | |
|---|---|---|---|---|---|---|---|---|
| Sample code | EHMA/MMA in feed^{a} | M/CTA^{a} | EHMA/MMA in copolymer^{a,}^{b} | M/CTA in cop^{a,}^{b} | yield (%) | start | Tg (°C) Tg | end |
| 1A | 70/30 | 100/4 | 70/30 | 100/2.3 | 62 | -6.3 | 7.2 | 17.4 |
| 1B | 70/30 | 100/6 | 70/30 | 100/3.6 | 70 | -11.9 | 2.5 | 10.3 |
| 1C | 70/30 | 100/8 | 70/30 | 100/4.5 | 77 | -16.5 | -6.5 | 5.3 |
| 2A | 67/33 | 100/4 | 67/33 | 100/2.5 | 40 | -6.1 | 5.9 | 19.0 |
| 2B | 67/33 | 100/6 | 66/34 | 100/3.5 | 73 | 12.2 | -0.9 | 9.6 |
| 2C | 67/33 | 100/8 | 67/33 | 100/4.4 | 73 | -16.5 | -8.4 | 4.4 |
| 3A | 63.5/36.5 | 100/4 | 63/37 | 100/2.4 | 51 | -1.8 | 9.5 | 24.7 |
| 3B | 63.5/36.5 | 100/6 | 65/35 | 100/3.8 | 71 | -12.4 | 0.5 | 10.8 |
| 3C | 63.5/36.5 | 100/8 | 63/37 | 100/4.5 | 64 | -18.2 | -10.4 | 6.8 |

| | | | | | | | | |
|---|---|---|---|---|---|---|---|---|
| a) in mol/mol, | | | | | | | | |
| b) determined by ¹H NMR after addition of TFAA. The amount of MAA incorporated in the polymer could not be determined. | | | | | | | | |

Of these polymers, the molecular weights were determined by GPC using tetrahydrfuran as a solvent and polystyrene with different molecular weights as calibration standards. The data of the results are shown in table 3.

**Table 3:**

| Molecular weights of polymers | | | |
|---|---|---|---|
| Sample code | M_{w} | Mₙ | M_{w}/Mₙ |
| 1A | 8.4 | 5.3 | 1.6 |
| 1B | 6.9 | 4.5 | 1.5 |
| 1C | 5.7 | 3.8 | 1.5 |
| 2A | 8.5 | 5.2 | 1.6 |
| 2B | 6.5 | 4.2 | 1.5 |
| 2C | 5.6 | 3.6 | 1.5 |
| 3A | 8.0 | 4.9 | 1.8 |
| 3B | 6.5 | 4.3 | 1.5 |
| 3C | 5.3 | 3.7 | 1.4 |

## Claims

1. A thermosensitive adhesive composition based on a copolymer comprising:
a) a first monomer chosen from the group of alkyl acrylates and alkyl methacrylates containing in the alkyl group 4 to 10 carbon atoms;
b) a second monomer chosen from the group of alkyl acrylates or methacrylates containing in the alkyl group 1 to 3 carbon atoms, acrylamides, methacrylamides and other unsaturated monomers; and
c) 1 to 15 mole% of acrylic acid, methacrylic acid and/or hydroxyethyl methacrylic acid,
which copolymer has a glass transition temperature between -25 and 20°C.

2. An adhesive composition according to claim 1, wherein the molar ratio between the first and the second monomer is between 1:1 and 3:1.

3. An adhesive composition according to claim 1 or 2, wherein the copolymer has a glass transition temperature between -15 and 10°C.

4. An adhesive composition according to any of the preceding claims, wherein the acrylic acid, methacrylic acid and/or hydroxyethyl methacrylic acid is present in an amount of 2 to 8 mole%.

5. An adhesive composition according to any of the preceding claims, wherein the first monomer is chosen from the group of n-butyl, n-pentyl, n-hexyl, isoheptyl, n-nonyl, n-decyl, isohexyl, 2-ethyloctyl, isooctyl and 2-ethylhexyl acrylates or methacrylates, and combinations thereof.

6. An adhesive composition according to any of the preceding claims, wherein the second monomer is chosen from the group of acrylamide, diacetone acrylamide, N-vinyl-2-pyrrolidone, vinyl acetate, N-isopropyl acrylamide, and methyl methacrylate, and combinations thereof.

7. An adhesive composition according to any of the preceding claims, wherein the copolymer has a weight average molecular weight of between 2000 and 25,000.

8. An adhesive composition according to any of the preceding claims in the form of an aqueous dispersion.

9. Use of an adhesive composition according to any of the preceding claims for adhering a prosthesis to human epithelium.

10. Use according to claim 9, wherein the prosthesis is a removable dental prosthesis.

11. A prosthesis comprising a surface to which an adhesive according to any of the claims 1-8 is applied.
